# EUROPEAN PATENT APPLICATION

(11) **EP 3 653 197 A1**
(43) Date of publication of application: **20.05.2020**
(21) Application number: 19212925.2
(22) Date of filing: 20.10.2010
(51) Int. Cl.: A61K 8/26, A61K 8/28, A61K 8/34, A61K 8/37, A61K 8/49, A61K 8/97, A61Q 15/00, A61K 8/31, A61K 8/365, A61K 8/9767, A61K 8/9789

(54) **ANTIPERSPIRANT THAT REDUCES/ELIMINATES YELLOWING ON CLOTHING**

(30) Priority: 20.10.2009 US 25323809 P; 15.12.2009 US 28640409 P
(62) Divisional of application: 10769131.3
(71) Applicant: Colgate-Palmolive Company, New York, NY 10022 (US)
(72) Inventor: GALE, Anne, Landing, NJ New Jersey 07850 (US); ADAMS, Richard, Piscataway, NJ New Jersey 08854 (US); JOHANSSON, Marie, Watchung,, NJ New Jersey 07069 (US); TINSLEY, Rebecca, North Brunswick, NJ New Jersey (US)
(74) Representative: Klaus, Stephan

(57) **Abstract**

The present application is directed to an antiperspirant composition that reduces or eliminates yellowing on clothing. It comprises a) a base

b) an antiperspirant active; and

c) at least one antioxidant chosen from butylated hydroxytoluene, pentaerythrityl tetra-di-t-butyl hydroxyhydrocinnamate, caffeine, and abies picea extract in an amount that is greater than an amount for stabilizing the antiperspirant composition.

## Description

### CROSS REFERENCE TO RELATED APPLICATIONS

This application claims priority to U.S. Provisional Patent Application Nos. 61/253,238, filed on 20 October 2009, and 61/286,404, filed on 15 December 2009, all of which are incorporated herein by reference.

### BACKGROUND

When using aluminum containing antiperspirants, the aluminum antiperspirant can contribute to yellow discoloration on clothing. This primarily occurs in the area of clothing that comes in contact with the area on the skin that contains the antiperspirant. While wearing the clothing, the antiperspirant can be transferred to the clothing. While aluminum alone does not cause yellowing, the aluminum in combination with sebum and/or sweat contribute to the yellowing. The aluminum helps bind the sebum and other particulate soils to clothing. The sebum and other particulate soils are oxidizable. Also, any iron metal that is present can contribute to yellowing.

In addition to the yellowing at the point of contact on clothing, the yellowing can be transferred to other areas of the clothing during laundering of the clothing. The combination of materials that contribute to yellowing can be redeposited to other areas of the clothing during laundering. The yellowing is more noticeable on white or light colored clothing, such as white t-shirts. For more information about discoloration on clothing, see Preventing Discoloration of Squalene-Soiled Cotton Fabrics with Antioxidants, by Yong-Seung Chi and S. Kay Obendorf, Journal of Surfactants and Detergents, Vol. 1, No. 4 (October 1998), pp. 523-527.

While one could reduce the level of yellowing by lowering the level of antiperspirant active in the antiperspirant, it is most likely expected that the clinical efficacy of the antiperspirant product would also be reduced because of the lower amount of antiperspirant active. Such a product would be expected to be undesirable to consumers because the product would not be effective at being an antiperspirant.

There is a consumer desire to reduce or eliminate yellowing of clothing while still maintaining the desired level of clinical efficacy.

### SUMMARY

An antiperspirant composition comprising (a) a base; (b) an antiperspirant active; and (c) at least one antioxidant chosen from butylated hydroxytoluene, pentaerythrityl tetra-di-t-butyl hydroxyhydrocinnamate, caffeine, and abies picea extract in an amount that is greater than an amount for stabilizing the antiperspirant composition.

### DETAILED DESCRIPTION

As used throughout, ranges are used as a shorthand for describing each and every value that is within the range. Any value within the range can be selected as the terminus of the range. In addition, all references cited herein are hereby incorporated by reference in their entireties. In the event of a conflict in a definition in the present disclosure and that of a cited reference, the present disclosure controls,

Unless otherwise specified, all percentages and amounts expressed herein and elsewhere in the specification should be understood to refer to percentages by weight. The amounts given are based on the active weight of the material.

The present invention is directed to an antiperspirant composition that reduces or eliminates the yellowing on clothing. The yellowing is most noticeable on lighter colored clothing, such as white T-shirts.

The antiperspirant compositions contain an antioxidant chosen from butylated hydroxytoluene (BHT), pentaerythrityl tetra-di-t-butyl hydroxyhydrocinnamate (Tinogard TT™ from Ciba/BASF), caffeine, and abies picea extract (GranLux™ AOX-G4 from Granula, which is a spruce knot extract in butylenes glycol) in an amount that is greater than an amount that is needed to stabilize the antiperspirant composition. The purpose of having the amount greater than the amount needed for the composition is to have the antioxidant available for reducing or eliminating yellow discoloration on clothing. The amount of antioxidant is at least 10% greater than the amount needed to stabilize the composition. For example, if 0.1 weight% based on the total weight of the composition is needed to stabilize, then at least 0.11 weight% would be used. In other embodiments, the amount is at least 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 325, 350, 375, 400, 425, 450, 475, or 500%. The amount of a specific antioxidant based on the weight of the composition will depend on the antioxidant used. Generally, the amount of BHT will be 0.05 to 0.5 weight%, the amount of pentaerythrityl tetra-di-t-butyl hydroxyhydrocinnamate (Tinogard TT™) will be 0.03 to 0.5 weight%, the amount of caffeine will be 0.1 to 0.5 weight%, and the amount of abies picea extract (GranLux™ AOX-G4) will be 1 to 3 weight%. The antioxidants can be used in combination with each being in their amounts listed above. In one embodiment, the antioxidant is a combination of butylated hydroxytoluene and pentaerythrityl tetra-di-t-butyl hydroxyhydrocinnamate. For caffeine, lower amounts are better. If the amount is too high, there can be an increase in yellowing.

In certain embodiments, the composition further includes citric acid. In certain embodiments, the citric acid is present in an amount of 0.01 to 0.1 weight% of the composition. In certain embodiments, the amount is at least 0.02, 0.03, 0.04, 0.05, 0.06, 0.07, 0.08, or 0.09 up to 0.1 weight%. In certain embodiments, the amount is 0.02 to 0.04 weight%.

During wearing, some of the antiperspirant composition that is applied to the skin is transferred to the clothing. By having a higher level of antioxidant, the antioxidant is available to the clothing to reduce or eliminate the yellowing. It has been noticed that not all antioxidants provide a reduction in yellowing. Some antioxidants, such as tocopherols (vitamin E) or mixtures of tocopherol (such as Oxynex™ K liquid, which contains PEG-8, tocopherol, ascorbyl palmitate, ascorbic acid, and citric acid), rosemary extract, white tea extract, or grapeseed extract actually increase the level of yellowing or do not lower the level of yellowing.

Antioxidants have been included in antiperspirant compositions. This purpose was for stabilizing the antiperspirant compositions themselves to prevent yellowing of the composition. The amount in these compositions is the minimum amount needed that provides stabilization to the composition. Because antioxidants add additional cost, and because an inclusion of a higher amount would not result in any additional benefit to the composition itself, antiperspirant products are designed to minimize the amount of antioxidant to save cost. The actual amount of antioxidant is dependent on the specific composition depending on the materials in the composition. Still, the level would be selected to be the minimum amount needed to achieve stabilization.

In one embodiment, the level of yellowing can additionally be reduced by lowering the amount of available iron. This can be achieved by using a low iron antiperspirant. Such low iron antiperspirant can be obtained from SummitReheis Antiperspirant Actives (Huguenot, New York). The level of iron is less than 100ppm. Typically, the amount of iron is 40 to 50ppm. The use of chelating agents, such as trisodium ethylenediamine disuccinate, methylglycinediacetic acid trisodium salt, or sodium phytate to remove iron can result in a slight increase in yellowing as compared to the same formula without the chelating agent. Even though a chelating agent increases the level of yellowing, it can still be included if the level of yellowing is below the desired level (see below). Generally, the amount of chelating agent is 0.03 to 0.15 weight%. In one embodiment, the resulting level of iron is less than 10 ppm of the composition. In another embodiment, the amount of iron is 20 to 40 ppm.

### Antiperspirant active materials

The composition includes an antiperspirant active. Any of the known aluminum containing antiperspirant active materials can be utilized in the composition. Antiperspirant actives include, but are not limited to, aluminum chlorohydrate, aluminum chloride, aluminum chlorohydrex polyethylene glycol, aluminum chlorohydrex propylene glycol, aluminum dichlorohydrate, aluminum dichlorohydrex polyethylene glycol, aluminum dichlorohydrex propylene glycol, aluminum sesquichlorohydrate, aluminum sesquichlorohydrate polyethylene glycol, aluminum sesquichlorohydrate propylene glycol, aluminum-zirconium octachlorohydrate, aluminum-zirconium octachlorohydrex gly, aluminum-zirconium pentachlorohydrate, aluminum-zirconium pentachlorohydrex gly, aluminum-zirconium tetrachlorohydrate, aluminum-zirconium tetrachlorohydrex gly, aluminum-zirconium trichlorohydrate, aluminum-zirconium trichlorohydrex gly, and combinations thereof. Generally, any of the Category I active antiperspirant ingredients, listed in the Food and Drug Administration's Monograph on Antiperspirant Drug Products for over-the-counter human use (Oct. 10, 1973) can be used (21 CFR 350.10). In one embodiment, the antiperspirant active is aluminum chlorohydrate. In another embodiment, the antiperspirant active is aluminum zirconium tetrachlorhydrex propylene glycol.

In other embodiments, the antiperspirant active is an aluminum salt and/or an aluminum-zirconium salt, such as those described above, that are further stabilized by betaine and a calcium salt. More information betaine and calcium salt stabilized antiperspirant salts can be found in U.S. Patent Application Publication No. 2006/0204463 to Tang et al. In other embodiments, the antiperspirant active, such as those described above, is selected to have a low metal to chloride ratio. Examples of these antiperspirant actives can be found in U.S. Patent No. 6,375,937 to Chopra et al. and in U.S. Patent Application Publication No. 2004/0109833 to Tang et al. In other embodiments, the type of salt of interest, an aluminum zirconium tetrasalt or octasalt free of glycine are used wherein aluminum zirconium salt is stabilized by Betaine and has a metal to chloride ratio of 0.9:1 to 1.3:1 (and in other embodiments of 0.9:1 to 1.2:1 or 0.9:1 to 1.1:1). For the tetrasalt, the Al/Zr atomic ratio can be 3.2:1 to 4,1:1.0 and the Betaine:zirconium mole ratio can be 0.2:1 to 3.0:1 (or in other embodiments of 0.4:1 to 1.5:1). Another salt that can be used is an aluminum chloride salt buffered by Betaine, wherein the salt has a metal to chloride ratio of 0.9:1 to 1.3:1 (and in other embodiments of 0.9:1 to 1.2:1 or 0.9:1 to 1.1:1). For the octasalt the Al/Zr atomic ratio is 6.2:1 to 10.0:1 and the Betaine:Zr mole ratio is 0.2:1 to 3.0:1 (or in other embodiments of 0.4:1 to 1.5:1). In one embodiment, in the case of a salt that contains zirconium, the Betaine is incorporated during the synthesis of the salt so as to maximize the stabilizing effect this ingredient has (especially on the zirconium species). Alternatively, it can be post added to a glycine-free salt along with additional active phase ingredients to form a Betaine stabilized active. Additionally, the antiperspirant active can be a calcium salt stabilized antiperspirant active. Examples of calcium salt stabilized antiperspirant actives can be found in U.S. Patent Application Publication No. 2006/0204463.

Examples of commercially available glycine-free low M:Cl ratio tetrasalts and octasalts include, but are not limited to, REZAL™ AZP 955 CPG and REZAL™ AZP 885 respectively (both from SummitReheis Antiperspirant Actives of Huguenot, New York). A more detailed description of making such commercially available salts can be found for example, in U.S. Patent Nos. 7,074,394 and 6,960,338. Further examples of making these types of salt complexes are described in U.S. Patent Application Publication No. 2004/0198998 and United States Patent No. 7,105,691.

Antiperspirant actives can be incorporated into compositions in amounts of 1 to 25 weight% (on an actives basis) of the final composition, but the amount used will depend on the formulation of the composition. Generally at lower levels the antiperspirant active material will not substantially reduce the flow of perspiration as effectively, but will reduce malodor, for example, by acting also as an antimicrobial material. In certain embodiments, the base antiperspirant material can be designed to more effectively deliver the antiperspirant to the skin. In these situations, the amount of antiperspirant can be lowered but still deliver the same level of efficacy as a product with higher levels of antiperspirant. For an example of a composition that provides the same clinical efficacy at a 10 weight% antiperspirant level as other compositions that have a 17 weight% antiperspirant level, see the hydrocarbon/hydrogenated soybean oil gelled formulation in U.S. Patent Application Publication No. 2008/0187504A1. In certain embodiments, the amount of antiperspirant active is less than 12 weight%. In other embodiments, the amount of antiperspirant active is 5 to 10 weight%, 6 to 10 weight%, 7 to 10 weight%, or 8 to 10 weight%. Base

The term base is used to encompass all other materials in an antiperspirant composition that is not the antiperspirant active. The antiperspirant composition can be in the form of a stick, a soft solid, a gel, a roll-on, or an aerosol.

In one embodiment, the composition is a solid stick or soft solid when at ambient room temperature of 25°C. The stick form is an example of a solid form, and the soft solid is a thickened form that may or may not be solid. The stick form can be distinguished from a soft solid in that, in a stick, the formulated product can retain its shape for extended time periods outside the package, the product not loosing its shape significantly (allowing for some shrinkage due to solvent evaporation). Adjustment of amounts of gelling or thickening agents can be used in order to form a soft solid or stick.

In one embodiment, the compression force of a stick product is at least 3000g. In other embodiments, the compression force is at least 4000g, at least 4500g, at least 5000g, at least 6000g, at least 7000g, at least 8000g, at least 9000g. In another embodiment, the compression force is 3500g to 10,000g.

In one embodiment, a stick product can provide a payout of 0.7 to 0.9g according to the payout test on the Payout, Glide, and Flakeoff Test Machine. As used in this specification, Payout, Glide, and Flakeoff Test Machine refers to the system described in WO2009/045557. In another embodiment, a stick product can provide a glide of 0.8 to 1.4g according to the glide test on the Payout, Glide, and Flakeoff Test Machine. In another embodiment, the stick product can provide a flakeoff of less that 25%. In other embodiments, the flake off is less than 20, 15, 10, or 5%. In other embodiments, the amount of flakeoff is 1 to 6%.

### Deodorant active materials

In certain embodiments, the composition may include any known deodorant active. Examples of deodorant actives include, but are not limited to, antimicrobial actives, alcohols, 2,4,4'-trichloro-2'-hydroxy diphenyl ether (Triclosan), benzethonium chloride, polyhexamethylene biguanides, triethylcitrate, 2-amino-2-methyl-1-propanol (AMP), cetyl-trimethylammomium bromide, cetyl pyridinium chloride, famesol (3,7,11-trimethyl-2,6,10-dodecatrien-1-ol), N-(4-chlorophenyl)-N'-(3,4-dichlorophenyl)urea (Triclocarban), silver halides, octoxyglycerin (Sensiva™ SC 50) and various zinc salts (for example, zinc ricinoleate), bactericides, and/or bacteriostats. The deodorant active can, illustratively, be included in the composition in an amount of 0-5%, or 0.01-1% by weight, of the total weight of the composition. Triclosan can, illustratively, be included in an amount of 0.05% to 0.5% by weight, of the total weight of the composition.

### Gelling Agents

Gelling agents can be included in antiperspirant products that typically contain gelling agents. Examples of gelling agents include, but are not limited to, waxes, esters of fatty acid and fatty alcohol, triglycerides, partially or fully hydrogenated soybean oil, partially or fully hydrogenated castor oil, other partial or fully hydrogenated plant oils, stearyl alcohol, or other cosmetically acceptable materials, which are solid or semi solid at room temperature and provide a consistency suitable for application to the skin.

In one embodiment, the gelling agent comprises a combination of hydrogenated soybean oil and a hydrocarbon of the formula CₙH₂ₙ₊₂, wherein n is 20 to 100, and the hydrocarbon is at least 90% linear. In this embodiment, the antiperspirant composition has a structure that provides a better delivery of the antiperspirant to the skin. Instead of using a typical 17 weight% level for the antiperspirant, a 10 weight% level can be used and still provide the same level of clinical wetness reduction as the 17 weight% level. By reducing the level of the antiperspirant in this embodiment, the amount of material that contributes to yellowing is reduced. This results in lower levels of yellowing.

In certain embodiments, the fully or partially hydrogenated soybean oil are those described in US2008/0187504A1 and US2008/0187503A1. The hydrogenated soybean oil from US2008/0187504A1 is almost, but not fully hydrogenated. The amount of hydrogenation is measured by the iodine value. The iodine value can be measured by ASTM D5554-95 (2006). The iodine value of the hydrogenated soybean oil used herein is greater than 0 to 20. In one embodiment, the iodine value is 1 to 5. The partially hydrogenated soybean oil from US2008/0187503A1 has a melting point that of -15°C (5°F) to 38°C (100°F). In another embodiment, the melting point is 26°C (80°F) to 38°C (100°F). To obtain the desired melting point, the oil can be partially hydrogenated or a blend of non-hydrogenated with partially or fully hydrogenated oils and/or waxes.

The partially or fully hydrogenated soybean oil is present in an amount up to 20% by weight of the composition. In another embodiment, the amount is up to 10% by weight. In one embodiment, the amount is at least 1, 2, 3, 4, 5, 6, 7, 8, or 9 % by weight. In another embodiment, the amount is less than 10, 9, 8, 7, 6, 5, 4, 3, 2, 1 % by weight. Any of the preceding minimum and maximum amounts can be combined to form any range of values.

The hydrocarbon is a hydrocarbon of the formula CₙH₂ₙ₊₂, wherein n is 20-100, and the hydrocarbon is at least 90% linear. In one embodiment, the hydrocarbon is a paraffin. In another embodiment, the hydrocarbon is polyethylene/polymethylene. An example of a polyethylene can be found in United States Patent No. 6,503,491. In another embodiment, the polyethylene has a weight average molecular weight of 300 to 3000 and a melting point of 50 to 129°C.

### Volatile silicone

Compositions according to the present invention can include a volatile silicone. In one embodiment, the volatile silicone is a volatile cyclic polydimethylsiloxane (cyclomethicone), e.g., cyclopentasiloxane. By volatile material it is meant that the material has a measurable vapor pressure at ambient temperature. Preferably, the volatile cyclic polydimethylsiloxane is cyclomethicone. Various types of cyclomethicones may be used. Illustratively, and not by way of limitation, the volatile silicones are one or more members selected from cyclic polydimethylsiloxanes such as those represented by Formula I: 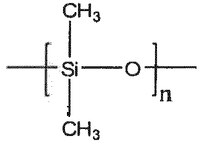 where n is an integer with a value of 3-7, particularly 5-6. Illustrative examples of suitable cyclomethicones are DC-345 and DC-245, manufactured by Dow Corning Corporation, Midland, MI. These types include a tetramer (octylmethylcyclotetrasiloxane) and a pentamer (decamethylcyclopentasiloxane). In one embodiment, the amount of volatile silicone in the composition is 5 to 70% by weight of the composition. In another embodiment, the amount is 25 to 45% by weight.

### Emollients

The composition can contain emollients in any desired amount to achieve a desired emollient effect. Emollients are known in the art and are used to impart a soothing effect on the skin. Non-volatile emollients are preferable in the present invention. Classes of non-volatile emollients include non-silicone and silicone emollients. Non-volatile, non-silicone emollients include C₁₂₋₁₅ alkyl benzoate. The non-volatile silicone material can be a polyethersiloxane, polyalkyarylsiloxane or polyethersiloxane copolymer. An illustrative non-volatile silicone material in the present invention is phenyl trimethicone. Non-limiting examples of emollients can be found in United States Patent No. 6,007,799. Examples include, but are not limited to, PPG-14 butyl ether, PPG-3 myristyl ether, stearyl alcohol, stearic acid, glyceryl monoricinoleate, isobutyl palmitate, glyceryl monostearate, isocetyl stearate, sulphated tallow, oleyl alcohol, propylene glycol, isopropyl laurate, mink oil, sorbitan stearate, cetyl alcohol, hydrogenated castor oil, stearyl stearate, hydrogenated soy glycerides, isopropyl isostearate, hexyl laurate, dimethyl brassylate, decyl oleate, diisopropyl adipate, n-dibutyl sebacate, diisopropyl sebacate, 2-ethyl hexyl palmitate, isononyl isononanoate, isodecyl isononanoate, isotridecyl isononanoate, 2-ethyl hexyl palmitate, 2-ethyl hexyl stearate, Di-(2-ethyl hexyl) adipate), Di-(2-ethyl hexyl) succinate, isopropyl myristate, isopropyl palmitate, isopropyl stearate, octacosanol, butyl stearate, glyceryl monostearate, polyethylene glycols, oleic acid, triethylene glycol, lanolin, castor oil, acetylated lanolin alcohols, acetylated lanolin, petrolatum, isopropyl ester of lanolin, fatty acids, mineral oils, butyl myristate, isostearic acid, palmitic acid, PEG-23 oleyl ether, olelyl oleate, isopropyl linoleate, cetyl lactate, lauryl lactate, myristyl lactate, quaternised hydroxy alkyl, aminogluconate, vegetable oils, isodecyl oleate, isostearyl neopentanoate, myristyl myristate, oleyl ethoxy myristate, diglycol stearate, ethylene glycol monostearate, myristyl stearate, isopropyl lanolate, paraffin waxes, glycyrrhizic acid, hydrocyethyl stearate amide.

The composition can additionally include ionizable inorganic salts. These ionizable salts are of the form MₐX_{b} where a=1, or 2 and b=1 or 2; M is a member chosen from Na⁺¹, Li⁺¹, K⁺¹, Mg⁺², Ca⁺², Sr⁺², and Zn⁺² and X is a member chosen chloride, bromide, iodide, citrate, gluconate, lactate, glycinate, glutamate, ascorbate, aspartate, nitrate, phosphate, hydrogenphosphate, dihydrogenphosphate, formate, maloneate, maleate, succinate, carbonate, bicarbonate, sulfate, and hydrogensulfate. In certain embodiments, the selected salts are chosen from NaCl and ZnCl₂. As will be appreciated by those skilled in the art, while it may be possible under certain circumstances to add a salt directly to a portion of the mixture during manufacturing, it is desired to add the salt as a mixture or solution of the salt in a carrier or solvent, particularly water. Of course various concentrations of the salt premix can be made.

The composition may also contain particulates, which include, but are not limited to, talc, mica, fragrance encapsulates, or hydrophobically modified starches, such as aluminum starch octenyl succinate (MACKADERM™ ASTRO-DRY™ from McIntyre Group Ltd.). If the composition is in a liquid form and dispensed through a roll-on applicator, the average particle size of the suspended material is sized so that it can pass through the application to prevent the ball applicator from malfunctioning. Usually, the average particle size does not exceed 150 microns.

In certain embodiments, the composition may also contain as an optional ingredient at least one malodor counteracting alpha, beta-unsaturated ester or mixtures of such materials. In certain embodiments, the level of malodor counteracting composition to deliver a perceivable odor control benefit when delivered from an antiperspirant and/or deodorant composition is 0.05 to 0.45 weight % based on the entire composition. The alpha, beta-unsaturated ester malodor counteracting materials are incorporated within the oil phase of an antiperspirant composition. Example of these malodor counteracting components can be found in U.S. Patent No. 6,610, 648 and U.S. Patent No. 6,495,097, which are incorporated herein only for their disclosure of the alpha, beta unsaturated esters. For example, in this invention the odor neutralizing alpha, beta unsaturated ester mixture demonstrates unexpected stability in antiperspirant compositions containing low metal:chloride (M:Cl) ratio salts free of glycine. Examples of the alpha, beta unsaturated ester can be found in WO2005/025523, which was filed in the United States as U.S. Application No. 10/571,488, both of which are incorporated herein by reference to the extent that they do not conflict with the disclosure in this specification.

Examples of the alpha, beta unsaturated ester include, but are not limited to,:
(1) 3-phenyl-2-propenoic acid alkyl esters wherein R¹ is a substituent on the benzene ring and is chosen from an alkyl, an alkoxy, an aryl, or a substituted aryl. In certain embodiments, R¹ is chosen from H, a C₁ to C₈ alkyl, a C₁ to C₈ alkoxy, or an aryl; and R² is a subsistent group replacing the carboxylic acid hydrogen to form the ester where R² has greater than 6 carbon atoms, an aryl, or a substituted aryl group, in certain embodiments R² is a C₆ to C₁₂ alkyl or is a benzyl group; and
(2) an ester of fumaric or maleic acid having linear ester carbon chains from 3-9 carbons, for example dihexyl fumarate;
(3) e-phenyl propenoic acid ester chosen from octyl methoxy cinnamate, phenylethyl cinnamate, benzyl cinnamate;
(4) an aliphatic unsaturated ester, such as dihexyl fumarate.

The composition may optionally further comprise absorbent materials such as corn starch, talc, clay, sodium polyacrylate and/or cotton fiber; and/or other materials such as fragrances, colorants, etc.

When water is present, for example in a liquid roll-on composition, the amount of water in the composition is the amount to make a 100% by weight composition after all of the materials, including any optional materials, are added to the composition. In certain embodiments, the amount of water is at least 20%, 30%, 40%, 50%, 60%, 70%, 80%, or 85% by weight of the composition.

The total solids of the composition is the amount of non-volatile materials in the composition. The percent solids is measured by a CEM Smart System moisture/solids analyzer which uses microwave energy to dry the samples. In one embodiment, the total solids is less than 25%. In another embodiment, the total solids is less than 20%.

In aerosol form, any known aerosol propellant can be used.

The compositions as provided herein are described and claimed with reference to their ingredients, as is usual in the art. As would be evident to one skilled in the art, ingredients may in some instances react with one another, so that the true composition of the final formulation may not correspond exactly to the ingredients listed. Thus, it should be understood that the invention extends to the product of the combination of the listed ingredients.

The compositions of the present invention may be manufactured using methods known in the art. Typically, the ingredients are combined and heated to melt the components (other than inert filler), and the melted components (together with particulate inert filler) are mixed. Desirably, volatile materials, such as the fragrance materials, are incorporated in the composition in the latter stages of the mixing cycle, in order to avoid volatilization thereof. After mixing, the molten composition can be poured directly into the dispensers, after which the compositions harden into a solid, and the container is capped to preserve the product until use.

In the following are set forth examples of the present invention. These examples are illustrative, and not limiting, of the present invention. In the following examples, all amounts are in percent of the total weight of the composition.

To make stick compositions, emollients are placed in a 600 ml beaker. The emollients are heated with stirring to 65°C. The gellants are added, and the mixture is heated to 82-85°C. The mixture is cooled to 80°C, and cyclomethicone, which is preheated to 70°C, is added. The mixture is cooled to 75°C and the antiperspirant is added. The temperature is increased to 80°C and held for 10 minutes, and the remaining ingredients are added and mixed for one minute. The mixture is poured into oval containers of the type used for antiperspirants/deodorants, and they are placed in a refrigerator at 4°C for 15 minutes. Cooling is completed at room temperature.

Compression is measured using a Texture Analyzer (model #TA-XT21 from Texture Technologies Corp) fitted with a 19 mm square end probe. The antiperspirant stick is removed from the barrel and placed in a hardness sample holder. The sample is positioned so that 2.54 cm (1 inch) of the sample, measured at edge of domed portion is exposed from the Compression Holder for the test. The cover on the hardness holder is closed, and the holder is positioned so that the blade will come in contact at the midpoint of the exposed sample. The instrument is set to run at 1.0 mm/s at a distance of 5.0 mm. The peak value of the compression curve is recorded as the stick hardness value in grams.

Color can be measured by the L* a* b* system established by the Commission Internationale d'Eclairage (CIE). (See for example, McClelland, D., Macworld® Photoshop®4 Bible, IDG Books Worldwide, Inc. 1997, pp. 157-184.)

In certain embodiments, compositions of the invention can achieve a delta b* value of 4 or less after 3 cycles of simulated wearing and washing according to the Simulated Wear and Wash test described below. In other embodiments, the delta b* is no more than 3.5, 3, 2.5, 2, 1.5, 1, or 0.5. In other embodiments, any of the preceding delta b* values can be obtained after 5, 6, 7, 8, 9, 10, 11, 12, 13, 14, 15, 20, or 25 cycles.

### Simulated Wear and Wash Test

Prepare swatches with product: Sebum swatches are obtained from Testfabrics, Inc - with 0.6g sebum drop-applied to #460 cotton interlock knit, 7.6 cm x 10.2 cm (3" x 4").
- Take three 7.6 cm x 15.2 cm (3" x 6") cotton knit swatches (cotton interlock #460 from Testafabrics, Inc.);
- Place a supporting foam square (4cm x 5cm, foamed polyethylene sheet) on the lab bench and lay the swatch over the top, centering the square in the bottom half of the swatch;
- Lay the plastic mask (template) over the support square and press down around the edges, raising the fabric over the support;
- Apply 0.23g - 0.27 g of product from the antiperspirant stick, spreading as evenly as possible. The treated area is 20cm² (4cm x 5cm);
- Place a sebum swatch on the lower half of this top swatch;
- Match the layers so that the applied active/product is directly against the sebum swatch;
- Place a plain 7.6 cm x 10.2 cm (3" x 4") swatch (cotton interlock #460 from Testafabrics, Inc.) over the sebum swatch;
- Turn the layers over;
- Pipette 1 mL of artificial eccrine sweat onto the back side of the middle swatch over the area that the stick was applied;
- After the sweat is soaked into the swatch, lay a plain 7.6 cm x 10.2 cm (3" x 4") swatch on top of the sweat;
- Holding the swatches together, flip them over again so that the layers are in this order (top to bottom):
   ∘ Plain
   ∘ Sebum
   ∘ Product
   ∘ Plain;
- Cut a piece of Parafilm® film to fit over the treated area 7.6 cm x 10.2 cm (3" x 4");
- Place this Parafilm® film over the swatches in the lower half, especially covering the product portion;
- Put the 5 layers on top of a PVC pipe with the Parafilm® film being the outermost layer;
- Wrap an elastic bandage around the swatches to keep them in place on the pipe, and secure the wrap with clips or rubber bands;
- The final setup from top to bottom is:
   ∘ Elastic bandage
   ∘ Parafilm® film
   ∘ Top Blank Swatch
   ∘ Sebum-Treated swatch
   ∘ Product/sweat swatch with the product side facing up
   ∘ Bottom Blank Swatch
   ∘ PVC Pipe.

Place swatches in oven:
- heat the treated swatches in an oven (LabLine instruments Model 7085)
- oven at 40.6°C (105°F) for 16-18 hrs (no humidity control in oven).

Laundry and dry:
- Unwrap the swatches and dispose of the Parafilm® film and sebum patches. Swatch groups may be placed in a open plastic bag in a dark place overnight before washing;
- Set the washer at "Medium" size, "Hot/Cold" (wash/rinse), "Normal";
- Adjust water temperature to 40.6°C ± 1°C(105°F ± 2 °F)
- After the machine has filled, put in 100ml of Dynamo detergent (regular formula) or 40ml of Dynamo detergent (2x formula), 2 white cotton towels and 4 white cotton t-shirts for ballast, and the swatches;
- After the wash is done, dry the whole load for 45 minutes on the "cotton/high heat" setting;
- Remove swatches from dryer, sort into product groups and place each grouping in a separate sealable plastic bag until color readings are performed.

Intensity readings using Minolta Chromameter CR-300, illuminant D65, set to read L-a-b values:
- Place swatches on a white backing (such as laser printer paper, about 10 sheets in a stack);
- Press "Calibrate" and measure against Ceramic tile;
- Measure L-a-b values of paper backing (as a reference only);
- Measure L-a-b values of blank area of swatch (3 points);
- Measure L-a-b values of yellowed area of swatch (3 points);
- Calculate delta L, delta a, delta b* (difference between value of treated area and untreated area of swatch) and delta E (overall color change).

For each 0.2 reduction in the delta b* value, there is a consumer noticeable difference in the level of yellowing.

The formulas below are used for making artificial sweat and sebum. They are made by mixing of the ingredients.

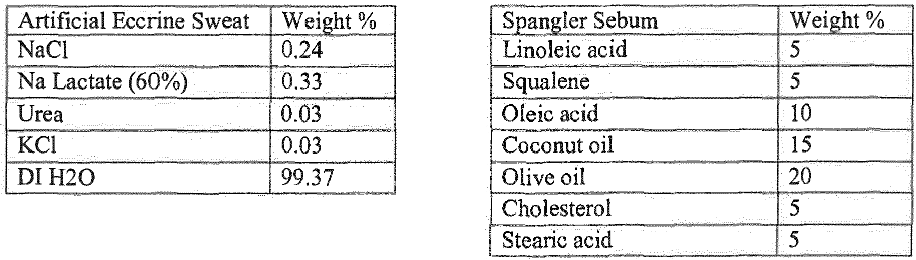

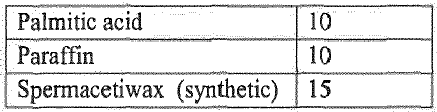

The antiperspirant composition can be used in a method to reduce and/or eliminate yellowing of clothing. The antiperspirant composition can be applied to a person in a typical application to an axillary area. The person then wears clothing. During wearing, some of the antiperspirant composition is transferred to the clothing in areas that are in contact with the area where the antiperspirant composition is applied. There is sufficient amount of antioxidant that is transferred by having a high enough concentration of antioxidant in the composition, a sufficient amount of the antiperspirant composition is transferred to the clothing, or a combination of both.

Body heat and higher temperatures during laundering or drying would cause the fatty acids present in sebum from the person to oxidize, which causes yellowing. The presence of antioxidants in the antiperspirant reduce or eliminate the oxidation, which reduces or eliminates yellowing.

The effect of this method can be increased when the level of antiperspirant is lowered. Having less aluminum reduces the amount of sebum that can be attached to clothing.

### SPECIFIC EMBODIMENTS

The invention is further described in the following examples. The examples are merely illustrative and do not in any way limit the scope of the invention as described and claimed.

Below are some examples of formulas for stick based antiperspirants that can be made according to the invention. The synthetic wax below is a hydrocarbon of the formula CₙH₂ₙ₊₂, wherein n is 20 to 100, and the hydrocarbon is at least 90% linear and the hydrogenated soybean oil has an iodine value up to 20.

| Ingredient | weight % |
|---|---|
| C12-15 Alkyl Benzoate | 4-15 |
| PEG-8 Distearate | 1-6 |
| Hydrogenated Soybean Oil | 0-10 |
| Synthetic Wax | 5-20 |
| tetra-di-t-butyl TT™) | 0.001-0.5 |
| 50% Citric Acid | 0.001-0.5 |
| Cyclomethicone DC 345 | 25-60 |
| BHT | 0.2 |
| Aluminum Zirconium Tetraclorohydrex Glycine | 1-24 |
| Fragrance | 0.5-2 |

| Ingredient | weight % |
|---|---|
| C12-15 Alkyl Benzoate | 4-15 |
| PEG-8 Distearate | 1-6 |
| Hydrogenated Soybean Oil | 0-10 |
| Synthetic Wax | 5-20 |
| pentaerythrityl tetra-di-t-butyl hydroxyhydrocinnamate (Tinogard TT™) | 0.04 |
| 50% Citric Acid | 0.1 |
| Cyclomethicone DC 345 | 25-60 |
| BHT | 0.1 |
| Aluminum Zirconium Tetraclorohydrex Glycine | 1-24 |
| Fragrance | 0.5-2 |

| Ingredient | weight % |
|---|---|
| C12-15 Alkyl. Benzoate | 4-15 |
| PEG-8 Distearate | 1-6 |
| Hydrogenated Soybean Oil | 0-10 |
| Synthetic Wax | 5-20 |
| pentaerythrityl tetra-di-t-butvl hydroxyhydrocinnamate (Tinogard TT™) | 0.02 |
| 50% Citric Acid | 0.5 |
| Cyclomethicone DC 345 | 25-60 |
| BHT | 0.5 |
| Aluminum Zirconium Tetraclorohydrex Glycine | 1-24 |
| Fragrance | 0.5-2 |

### EXAMPLE A

The following is the Base Formula used in the studies below. In the studies below, additional materials are included. When they are included, the amounts of the materials are adjusted so that the composition adds to 100 weight%.

| | weight % |
|---|---|
| C12-15 Alkyl Benzoate | 14.94 |
| PEG-8 Distearate | 4.00 |
| Hydrogenated Soybean Oil | 3.50 |
| Synthetic Wax | 13.00 |
| pentaerythrityl tetra-di-t-butyl hydroxyhydrocinnamate (Tinogard TT™) | 0.02 |
| 50% Citric Acid | 0.04 |
| Cyclomethicone DC 345 | 48.63 |
| Aluminum Zirconium Tetraclorohydrex Glycine | 14.12 |
| Perfume | 1.75 |
| | 100.00 |

The above formula was prepared with additional materials listed in the studies below and compared against commercially available products. In each of the comparisons, the products were used in the Simulated Wear and Wash test described above. They were run through 3 cycles of the wear and wash test before being tested. The testing for delta b* occurred after the completion of the third cycle. In some studies, they were tested after an additional time of sitting at room temperature (about 23°C) for 1 week or 1 month (as indicated below). The competitive antiperspirant products were purchased in the United States.

The results from one study are not comparable to results from other studies. The water used in the Wear and Wash test was from the municipal water supply. There could be variances in the chlorination levels of the water, or there could be quality variations in the water, such as after recent rain.

**Study 1**

| | Mennen SpeedStick ™ 24/7 Fresh Rush antiperspirant | Base Formula | Sure Invisible Solid antiperspirant |
|---|---|---|---|
| delta b* | 3.89 | 3.04 | 5.89 |

**Study 2**

| | Mennen SpeedStick ™ 24/7 Fresh Rush antiperspirant | Base Formula |
|---|---|---|
| delta b* | 7.1 | 5.9 |

**Study 3**

| | Base Formula | Base Formula + 0.1% BHT | Base Formula + 0.25% vitamin E acetate | Base Formula with doubled amounts of pentaerythrityl tetra-di-t-butyl hydroxyhydrocinnamate (Tinogard TT™) and citric acid |
|---|---|---|---|---|
| delta b* | 4.10 | 2.80 | 6.07 | 3.35 |
| delta b* + 1 month | 12.7 | 4.45 | 13.44 | 10.01 |

**Study 4**

| | Mennen SpeedStick ™ 24/7 Fresh Rush antiperspirant | Base Formula +0.1% BHT | Secret Invisible Solid antiperspirant | Dove Ultimate Beauty Care antiperspirant | Secret Flawless antiperspirant |
|---|---|---|---|---|---|
| delta b* | 2.42 | 1.96 | 2.64 | 2.49 | 2.99 |
| delta b* + 1 week | 2.57 | 2.01 | 2.97 | 2.79 | 3.55 |

**Study 5**

| | Mennen SpeedStick ™ 24/7 Fresh Rush antiperspirant | Base Formula +0.1% BHT | Old Spice Ever Clear antiperspirant | Old Spice Red Zone antiperspirant |
|---|---|---|---|---|
| delta b* | 33.17 | 2.54 | 5.31 | 2.92 |

**Study 6**

| | Mennen SpeedStick ™ 24/7 Fresh Rush antiperspirant | Base Formula + 0.1% abies picea extract (GranLux™ AOX-G4) | Base Formula + 0.15% sodium phytate | Prototype + 1% pentaerythrityl tetra-di-t-butyl hydroxyhydrocinnamate (Tinogard TT™) |
|---|---|---|---|---|
| delta b* | 2.13 | 1.85 | 1.99 | 1.64 |

**Study 7**

| | Mennen SpeedStick ™ 24/7 Fresh Rush antiperspirant | Base Formula +0.1% methylglycinediacetic acid trisodium salt (Trilon M™ from BASF) |
|---|---|---|
| delta b* | 2.20 | 2.06 |

**Study 8**

| | Mennen SpeedStick ™ 24/7 Fresh Rush antipespirant | Base Formula + 0.15% trisodium ethylenediamine disuccinate(Natrlquest E-30™) | Base Formula + 0.1% BHT +0.04% pentaerythrityl tetra-di-t-butyl hydroxyhydrocinnamate (Tinogard TT™) | Base Formula + 0.25% tocopherol |
|---|---|---|---|---|
| delta b* | 2.73 | 3.05 | 2.04 | 4.63 |

### EXAMPLE B

In this example, caffeine is evaluated as an antioxidant in an emulsion composition. The formulas are:

| Material | Weight % | | |
|---|---|---|---|
| | No AP | With ZAG | With ZAG & Caffeine |
| Steareth-2 | 3 | 3 | 3 |
| Steareth-20 | 1.5 | 1.5 | 1.5 |
| PEG-15 | 2 | 2 | 2 |
| Water | Q.S. | Q.S. | Q.S. |
| Aluminum Zirconium Tetraclorohydrex Glycine (ZAG) | 0 | 22 | 22 |
| Caffeine | 0 | 0 | 0.2 |

It is prepared by melting of the oil ingredients and mixing with the water. ZAG and caffeine are added after the formation of the emulsion.

The products along with Mennen SpeedStick™ 24/7 Fresh Rush antiperspirant were used in the Simulated Wear and Wash test described above. They were run through 3 cycles of the wear and wash test before being tested. The testing for delta b* occurred after the completion of the third cycle. After the 3 cycles, the samples were stored in a dark drawer at room temperature for one month and retested. They were returned to the dark drawer at room temperature for a second month and retested. Additionally, delta E (1976 standard) was measured along with delta b*. Delta E is ΔE = ((L₁-L₂)² + (a₁-a₂)² + (b₁-b₂)²)^{1/2}. The results are shown in the table below.

| delta b* values for the samples | 3^{rd} cycle | 1 month | 2 months |
|---|---|---|---|
| Mennen SpeedStick ™ 24/7 Fresh Rush antiperspirant | 1.67 | 4.86 | 14.06 |
| No Antiperspirant | 0.01 | 3.41 | 4.61 |
| With 22 weight % ZAG | 1.28 | 8.30 | 9.46 |
| With 22 weight% ZAG and 0.2weight% caffeine | 0.92 | 5.83 | 6.20 |

| delta E values for the samples | 3^{rd} cycle | 1 month | 2 months |
|---|---|---|---|
| Mennen SpeedStick ™ 24/7 Fresh Rush antiperspirant | 4.34 | 7.62 | 22.87 |
| No Antiperspirant | 0.82 | 3.62 | 4.87 |
| With 22 weight % ZAG | 1.70 | 8.78 | 9.92 |
| With 22 weight% ZAG and 0.2weight% caffeine | 1.16 | 6.18 | 6.49 |

It can be seen that the addition of caffeine to the composition with ZAG reduced the level of yellowing as measured by delta b*.

## Claims

1. An antiperspirant composition comprising:
a) a base;
b) an antiperspirant active; and
c) at least one antioxidant chosen from butylated hydroxytoluene, pentaerythrityl tetra-di-t-butyl hydroxyhydrocinnamate, caffeine, and abies picea extract in an amount that is greater than an amount for stabilizing the antiperspirant composition.

2. The composition of claim 1, wherein the antioxidant is a combination of butylated hydroxytoluene and pentaerythrityl tetra-di-t-butyl hydroxyhydrocinnamate.

3. The composition of claim 1, wherein the antioxidant comprises caffeine.

4. The composition of any preceding claim further comprising citric acid.

5. The composition of any preceding claim, wherein the amount of the antioxidant is at least 10% greater than the amount for stabilizing the antiperspirant composition.

6. The composition of any preceding claim, wherein the antiperspirant active is present in an amount of 10 weight% or less of the composition.

7. The composition of any preceding claim further comprising a chelating agent.

8. The composition of any preceding claim, wherein the composition has less than 10 ppm of iron.

9. The composition of any preceding claim, wherein the antiperspirant active before being combined in the composition has less than 20 ppm iron.

10. The composition of any preceding claim, wherein the antiperspirant composition is **characterized by** at least one of:
a) a payout of 0.7 to 0.9g;
b) a glide of 0.8 to 1.4g; and
c) a flakeoff of less than 25%.

11. The composition of any preceding claim, wherein the composition is a stick and the base comprises a gelling agent comprising a hydrocarbon of the formula CₙH₂ₙ₊₂, wherein n is 20 to 100, and the hydrocarbon is at least 90% linear and a hydrogenated soybean oil with an iodine value up to 20.

12. A method of reducing or eliminating yellowing on clothing comprising:
a) applying to a person an antiperspirant composition comprising:
i) a base, and
ii) at least one antioxidant chosen from butylated hydroxytoluene, pentaerythrityl tetra-di-t-butyl hydroxyhydrocinnamate, caffeine, and abies picea extract in an amount that is greater than an amount for stabilizing the personal care composition; and
b) wearing an article of clothing over the area where the antiperspirant composition was applied,
wherein an amount of antioxidant that is transferred onto clothing from the person during wearing of the clothing is sufficient to reduce or eliminate yellowing on the clothing.

13. The method of claim 12, wherein the antioxidant is a combination of butylated hydroxytoluene and pentaerythrityl tetra-di-t-butyl hydroxyhydrocinnamate.

14. The method of claim 13, wherein the antioxidant comprises caffeine.

15. The method of any of claims 12 to 14 further comprising citric acid.

16. The method of any of claims 12 to 15, wherein the amount of the antioxidant is at least 10% greater than the amount for stabilizing the antiperspirant composition.

17. The method of any of claims 12 to 16, wherein the antiperspirant active is present in an amount of 10 weight% or less of the composition.

18. The method of any of claims 12 to 17 further comprising a chelating agent.

19. The method of any of claims 12 to 18, wherein the composition has less than 10 ppm of iron.

20. The method of any of claims 12 to 19, wherein the antiperspirant active before being combined in the composition has less than 20 ppm iron.

21. The method of any of claims 12 to 20, wherein the antiperspirant composition is **characterized by** at least one of:
a) a payout of 0.7 to 0.9g;
b) a glide of 0.8 to 1.4g; and
c) a flakeoff of less than 25%.

22. The method of any of claims 12 to 21, wherein the composition is a stick and the base comprises a gelling agent comprising a hydrocarbon of the formula CₙH₂ₙ₊₂, wherein n is 20 to 100, and the hydrocarbon is at least 90% linear and a hydrogenated soybean oil with an iodine value up to 20.
